# EUROPEAN PATENT SPECIFICATION

(11) **EP 2 560 476 B1**
(45) Date of publication and mention of the grant of the patent: **13.12.2017**
(21) Application number: 11772786.7
(22) Date of filing: 22.04.2011
(51) Int. Cl.: A01H 5/00, A01H 5/10, C12N 5/04, A01N 63/02, C12N 15/82

(54) **COMBINATIONS INCLUDING CRY34AB/35AB AND CRY3BA PROTEINS TO PREVENT DEVELOPMENT OF RESISTANCE IN CORN ROOTWORMS (DIABROTICA SPP.)**
KOMBINATIONEN MIT CRY34AB/35AB- UND CRY3AA-PROTEINEN ZUR VERHINDERUNG DER ENTWICKLUNG EINES WIDERSTANDS IN MAISWURZELBOHRERN (DIABROTICA SPP.)
ASSOCIATIONS COMPRENANT DES PROTÉINES CRY34AB/35AB DESTINÉES À PRÉVENIR LE DÉVELOPPEMENT D'UNE RÉSISTANCE CHEZ LA CHRYSOMÈLE DU MAÏS (DIABROTICA SPP.)

(30) Priority: 20.04.2011 US 477447 P; 15.04.2011 US 476005 P; 30.09.2010 US 388273 P; 23.04.2010 US 327240 P
(43) Date of publication of application: 27.02.2013
(73) Proprietor: Dow AgroSciences LLC, Indianapolis, IN 46268 (US)
(72) Inventor: NARVA, Kenneth, E., Zionsville, IN 46077 (US); MEADE, Thomas, Zionsville, IN 46077 (US); FENCIL, Kristin, J., Indianapolis, IN (US); LI, Huarong, Zionsville, IN 46077 (US); HEY, Timothy, D., Zionsville, IN 46077 (US); WOOSLEY, Aaron, T., Fishers, IN 46038 (US); OLSON, Monica, Britt, Lebanon, IN 46052 (US)
(74) Representative: Krauss, Jan
(86) International application number: PCT/US2011/033618
(87) International publication number: WO 2011/133892

(56) References cited:
- US-A1- 2008 256 669
- US-A1- 2009 300 980
- KAISER-ALEXNAT ET AL: "Protease activities in the midgut of Western corn rootworm (Diabrotica virgifera virgifera LeConte)", JOURNAL OF INVERTEBRATE PATHOLOGY, SAN DIEGO, CA, US, vol. 100, no. 3, 2 March 2009 (2009-03-02) , pages 169-174, XP026075646, ISSN: 0022-2011, DOI: 10.1016/J.JIP.2009.01.003 [retrieved on 2009-01-25]
- SWAN CHRISTOPHER M ET AL: "Processing of transgenic crop residues in stream ecosystems", JOURNAL OF APPLIED ECOLOGY, vol. 46, no. 6, December 2009 (2009-12), pages 1304-1313, XP002712064,
- Kranthi K. R.: "INSECTICIDE RESISTANCE MANAGEMENT IN COTTON TO ENHANCE PRODUCTIVITY", , 15 December 2007 (2007-12-15), XP002712065, Retrieved from the Internet: URL:http://www.cicr.org.in/pdf/ELS/prot1.p df [retrieved on 2013-08-30]
- LI HUARONG ET AL: "Bacillus thuringiensis Cry34Ab1/Cry35Ab1 Interactions with Western Corn Rootworm Midgut Membrane Binding Sites", PLOS ONE, vol. 8, no. 1, January 2013 (2013-01), XP002712066,
- GATEHOUSE: 'Biotechnological prospects for engineering insect-resistant plants.' PLANT PHYSIOL. vol. 146, no. 3, March 2008, pages 881 - 887, XP002532323
- BRAVO ET AL.: 'How to cope with insect resistance to Bt toxins?' TRENDS BIOTECHNOL. vol. 26, no. 10, October 2008, pages 573 - 579, XP025406825
- ROUSH: 'Bt-transgenic crops: just another pretty insecticide or a chance for a new start in resistance management?' PESTICIDE SCIENCE vol. 51, no. 3, November 1997, pages 328 - 334, XP001196930
- BATES ET AL.: 'Insect resistance management in GM crops: past, present and future.' NAT BIOTECHNOL. vol. 23, no. 1, 2005, pages 57 - 62, XP055000762

## Description

### BACKGROUND

Humans grow corn for food and energy applications. Corn is an important crop. It is an important source of food, food products, and animal feed in many areas of the world. Insects eat and damage plants and thereby undermine these human efforts. Billions of dollars are spent each year to control insect pests and additional billions are lost to the damage they inflict.

Damage caused by insect pests is a major factor in the loss of the world's corn crops, despite the use of protective measures such as chemical pesticides. In view of this, insect resistance has been genetically engineered into crops such as corn in order to control insect damage and to reduce the need for traditional chemical pesticides.

Over 10 million acres of U.S. corn are infested with corn rootworm species complex each year. The corn rootworm species complex includes the northern corn rootworm (Diabrotica barberi), the southern corn rootworm (D. undecimpunctata howardi), and the western corn rootworm (D. virgifera virgifera). (Other species include Diabrotica virgifera zeae (Mexican corn rootworm), Diabrotica balteata (Brazilian corn rootworm), and Brazilian corn rootworm complex (Diabrotica viridula and Diabrotica speciosa).)

The soil-dwelling larvae of these Diabrotica species feed on the root of the corn plant, causing lodging. Lodging eventually reduces corn yield and often results in death of the plant. By feeding on cornsilks, the adult beetles reduce pollination and, therefore, detrimentally affect the yield of corn per plant. In addition, adults and larvae of the genus Diabrotica attack cucurbit crops (cucumbers, melons, squash, etc.) and many vegetable and field crops in commercial production as well as those being grown in home gardens.

Synthetic organic chemical insecticides have been the primary tools used to control insect pests but biological insecticides, such as the insecticidal proteins derived from *Bacillus thuringiensis* (*Bt*)*,* have played an important role in some areas. The ability to produce insect-resistant plants through transformation with *Bt* insecticidal protein genes has revolutionized modem agriculture and heightened the importance and value of insecticidal proteins and their genes.

Insecticidal crystal proteins from some strains of Bacillus thuringiensis (B.t.) are well-known in the art. See, e.g., Hofte et al., Microbial Reviews, Vol. 53, No. 2, pp. 242-255 (1989). These proteins are typically produced by the bacteria as approximately 130 kDa protoxins that are then cleaved by proteases in the insect midgut, after ingestion by the insect, to yield a roughly 60 kDa core toxin. These proteins are known as crystal proteins because distinct crystalline inclusions can be observed with spores in some strains of B.t. These crystalline inclusions are often composed of several distinct proteins.

One group of genes which have been utilized for the production of transgenic insect resistant crops are the delta-endotoxins from Bacillus thuringiensis (B.t.). Delta-endotoxins have been successfully expressed in crop plants such as cotton, potatoes, rice, sunflower, as well as corn, and have proven to provide excellent control over insect pests. (Perlak, F. J et al. (1990) Bio/Technology 8, 939-943; Perlak, F. J. et al. (1993) Plant Mol. Biol. 22: 313-321; Fujimoto H. et al. (1993) Bio/Technology 11: 1151-1155; Tu et al. (2000) Nature Biotechnology 18:1101-1104; PCT publication number WO 01/13731; and Bing J W et al. (2000) Efficacy of Cry IF Transgenic Maize, 14th Biennial International Plant Resistance to Insects Workshop, Fort Collins, Colo.)

Several *Bt* proteins have been used to create the insect-resistant transgenic plants that have been successfully registered and commercialized to date. These include Cry1Ab, Cry1Ac, Cry1F, Cry3Aa, and Cry3Bb in corn, Cry1Ac and Cry2Ab in cotton, and Cry3A in potato. There is also SMART STAX in corn, which comprises Cry1A.105 and Cry2Ab and furthermore Cry34Ab/Cry35Ab and Cry3Bb, see US 2009/0300980.

The commercial products expressing these proteins express a single protein except in cases where the combined insecticidal spectrum of 2 proteins is desired (*e.g.,* Cry1Ab and Cry3Bb in corn combined to provide resistance to lepidopteran pests and rootworm, respectively) or where the independent action of the proteins makes them useful as a tool for delaying the development of resistance in susceptible insect populations (*e.g.,* Cry1Ac and Cry2Ab in cotton combined to provide resistance management for tobacco budworm).

Some of the qualities of insect-resistant transgenic plants that have led to rapid and widespread adoption of this technology also give rise to the concern that pest populations will develop resistance to the insecticidal proteins produced by these plants. Several strategies have been suggested for preserving the utility of *Bt*-based insect resistance traits which include deploying proteins at a high dose in combination with a refuge, and alternation with, or co-deployment of, different toxins (McGaughey et al. (1998), "B.t. Resistance Management," Nature Biotechnol. 16:144-146).

The proteins selected for use in an Insect Resistance Management (IRM) stack should be active such that resistance developed to one protein does not confer resistance to the second protein (*i.e.,* there is not cross resistance to the proteins). If, for example, a pest population selected for resistance to "Protein A" is sensitive to "Protein B", one would conclude that there is not cross resistance and that a combination of Protein A and Protein B would be effective in delaying resistance to Protein A alone.

In the absence of resistant insect populations, assessments can be made based on other characteristics presumed to be related to cross-resistance potential. The utility of receptor-mediated binding in identifying insecticidal proteins likely to not exhibit cross resistance has been suggested (van Mellaert *et al.* 1999). The key predictor of lack of cross resistance inherent in this approach is that the insecticidal proteins do not compete for receptors in a sensitive insect species.

In the event that two *Bt* toxins compete for the same receptor, then if that receptor mutates in that insect so that one of the toxins no longer binds to that receptor and thus is no longer insecticidal against the insect, it might be the case that the insect will also be resistant to the second toxin (which competitively bound to the same receptor). That is, the insect is said to be cross-resistant to both *Bt* toxins. However, if two toxins bind to two different receptors, this could be an indication that the insect would not be simultaneously resistant to those two toxins.

A relatively newer insecticidal protein system was discovered in Bacillus thuringiensis as disclosed in WO 97/40162. This system comprises two proteins -- one of approximately 15 kDa and the other of about 45 kDa. See also U.S. Pat. Nos. 6,083,499 and 6,127,180. These proteins have now been assigned to their own classes, and accordingly received the Cry designations of Cry34 and Cry35, respectively. See Crickmore et al. website (biols.susx.ac.uk/home/Neil_Crickmore/Bt/). Many other related proteins of this type of system have now been disclosed. See e.g. U.S. Pat. No. 6,372,480; WO 01/14417; and WO 00/66742. Plant-optimized genes that encode such proteins, wherein the genes are engineered to use codons for optimized expression in plants, have also been disclosed. See e.g. U.S. Pat. No. 6,218,188.

The exact mode of action of the Cry34/35 system has yet to be determined, but it appears to form pores in membranes of insect gut cells. See Moellenbeck et al., Nature Biotechnology, vol. 19, p. 668 (July 2001); Masson et al., Biochemistry, 43 (12349-12357) (2004). The exact mechanism of action remains unclear despite 3D atomic coordinates and crystal structures being known for a Cry34 and a Cry35 protein. *See* U.S. Patent Nos. 7,524,810 and 7,309,785. For example, it is unclear if one or both of these proteins bind a typical type of receptor, such as an alkaline phosphatase or an aminopeptidase.

Furthermore, because there are different mechanisms by which an insect can develop resistance to a Cry protein (such as by altered glycosylation of the receptor [see Jurat-Fuentes et al. (2002) 68 AEM 5711-5717], by removal of the receptor protein [see Lee et al. (1995) 61 AEM 3836-3842], by mutating the receptor, or by other mechanisms [see Heckel et al., J. Inv. Pathol. 95 (2007) 192-197]), it was impossible to *a priori* predict whether there would be cross-resistance between Cry34/35 and other Cry proteins. Predicting competitive binding for the Cry34/35 system is also further complicated by the fact that two proteins are involved in the Cry34/35 binary system. Again, it is unclear if and how these proteins effectively bind the insect gut / gut cells, and if and how they interact with or bind with each other.

Other options for controlling coleopterans include the following proteins: Cry3Bb, Cry3C, Cry6B, ET29, ET33 with ET34, TIC407, TIC435, TIC417, TIC901, TIC1201, ET29 with TIC810, ET70, ET76 with ET80, TIC851, and others. RNAi approaches have also been proposed. *See e.g.* Baum et al., Nature Biotechnology, vol. 25, no. 11 (Nov. 2007) pp. 1322-1326.

### BRIEF SUMMARY

The subject invention relates in part to Cry34Ab/35Ab in combination with Cry3Ba. The subject invention relates in part to the surprising discovery that Cry34Ab/Cry35Ab and Cry3Ba are useful for preventing development of resistance (to either insecticidal protein system alone) by a corn rootworm (*Diabrotica* spp.) population. As one skilled in the art will recognize with the benefit of this disclosure, plants producing these insecticidal Cry proteins will be useful to mitigate concern that a corn rootworm population could develop that would be resistant to either of these insecticidal protein systems alone.

The subject invention is supported in part by the discovery that components of these Cry protein systems do not compete with each other for binding corn rootworm gut receptors.

The subject invention also relates in part to triple stacks or "pyramids" of three (or more) toxin systems, with Cry34Ab/Cry35Ab and Cry3Ba being the base pair. Thus, plants (and acreage planted with such plants) that produce these two insecticidal protein systems are included within the scope of the subject invention.

### BRIEF DESCRIPTION OF THE FIGURES

The detailed description of the drawings particularly refers to the accompanying figures in which:
**Figure 1A****.** Binding of ¹²⁵I-Cry35Ab1 as a function of input radio-labeled Cry toxins to BBMV prepared from western corn rootworm larvae. Specific binding =total binding - non-specific binding, error bar=SEM (standard error of mean).
**Figure 1B****.** Binding of ¹²⁵I-Cry3BAa1 as a function of input radio-labeled Cry toxins to BBMV prepared from western corn rootworm larvae. Specific binding =total binding - non-specific binding, error bar=SEM (standard error of mean).
**Figure 2****.** Binding of ¹²⁵I-Cry35Ab1 to BBMV prepared from western corn rootworm larvae at different concentrations of non-labeled competitor (log 0.1=-1.0, log10=1.0, log100=2.0, log1,000=3.0).
**Figure 3A****.** Percent binding of ¹²⁵I-Cry35Ab1 to BBMV prepared from western corn rootworm larvae in absence of Cry34Ab1.
**Figure 3B****.** Percent binding of ¹²⁵I-Cry35Ab1 to BBMV prepared from western corn rootworm larvae in presence of Cry34Ab1.

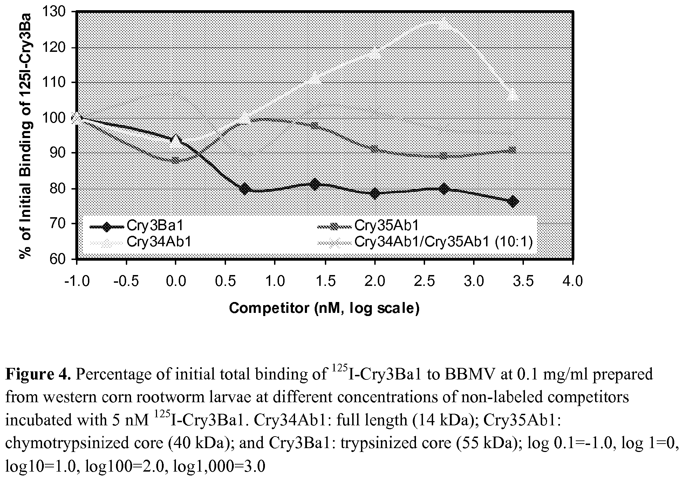

**Figure 4****.** Percent binding of ¹²⁵I-Cry3Ba1 to BBMV prepared from western corn rootworm larvae in presence of various concentrations of varying non-labeled competitors.

### BRIEF DESCRIPTION OF THE SEQUENCES

SEQ ID NO:1: Full length, native Cry35Ab1 protein sequence.
SEQ ID NO:2: Chymotrypsin-truncated Cry35Ab1 core protein sequence.
SEQ ID NO:3: Full length, native Cry3Ba1 protein sequence.
SEQ ID NO:4: Cry3Ba1 trypsin core protein sequence.
SEQ ID NO:5: Full length, native Cry34Ab1 protein sequence.

### DETAILED DESCRIPTION

Sequences for the Cry34Ab/35Ab protein are obtainable from *Bacillus thruingiensis* isolate PS149B1, for example. For other genes, protein sequences, and source isolates for use according to the subject invention, see the Crickmore et al. website (lifesci.sussex.ac.uk/home/Neil_Crickmore/ Bt/intro.html), for example.

The subject invention includes the use of Cry34Ab/35Ab insecticidal proteins in combination with a Cry3Ba toxin to protect corn from damage and yield loss caused by corn rootworm feeding by corn rootworm populations that might develop resistance to either of these Cry protein systems alone (without the other).

The subject invention thus teaches Insect Resistance Management (IRM) stacks to prevent the development of resistance by corn rootworm to Cry3Ba and/or Cry34Ab/35Ab.

The present disclosure provides compositions for controlling rootworm pests comprising cells that produce a Cry3Ba toxin protein and a Cry34Ab/35Ab toxin system.

The invention further comprises a host transformed to produce both a Cry3Ba protein and a Cry34Ab/35Ab binary toxin, wherein said host is a microorganism or a plant cell.

It is additionally intended that the invention provides a method of controlling rootworm pests comprising contacting said pests or the environment of said pests with an effective amount of a composition that contains a Cry3Ba protein and further contains a Cry34Ab/35Ab binary toxin.

An embodiment of the invention comprises a maize plant comprising a plant-expressible gene encoding a Cry34Ab/35Ab binary toxin and a plant-expressible gene encoding a Cry3Ba protein, and seed of such a plant.

A further embodiment of the invention comprises a maize plant wherein a plant-expressible gene encoding a Cry34Ab/35Ab binary toxin and a plant-expressible gene encoding a Cry3Ba protein have been introgressed into said maize plant, and seed of such a plant.

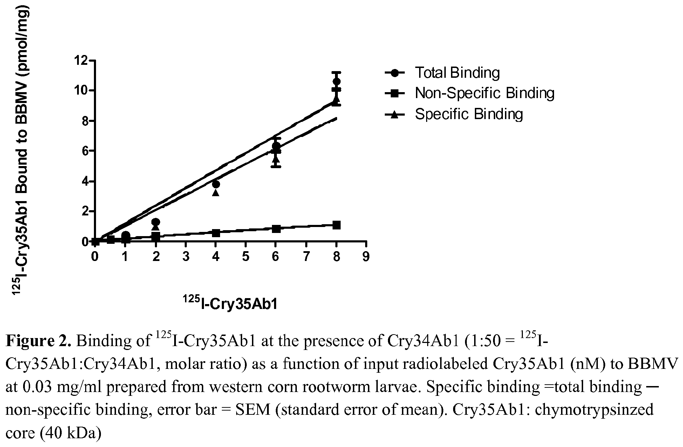

As described in the Examples, competitive receptor binding studies using radiolabeled Cry35Ab core toxin protein show that the Cry3Ba core toxin protein does not compete for binding in CRW insect tissue samples to which Cry35Ab binds. *See* **Figure 2****.** These results indicate that the combination of Cry3Ba and Cry34Ab/35Ab proteins is an effective means to mitigate the development of resistance in CRW populations to either protein system alone.

Thus, based in part on the data described above and elsewhere herein, Cry34Ab/35Ab and Cry3Ba proteins can be used to produce IRM combinations for prevention or mitigation of resistance development by CRW. Other proteins can be added to this combination to expand insect-control spectrum, for example. The subject combination (of Cry34Ab/35Ab and Cry3Ba proteins) can also be used in some preferred "triple stacks" or "pyramids" in combination with yet another protein for controlling rootworms, such as Cry3Aa and/or Cry6Aa; such additional combinations would thus provide multiple modes of action against a rootworm. RNAi against rootworms is a still further option. *See e.g.* Baum et al., Nature Biotechnology, vol. 25, no. 11 (Nov. 2007) pp. 1322-1326.

In light of the disclosure of USSN 61/327,240 (filed April 23, 2010) relating to combinations of Cry34Ab/35Ab and Cry3Aa proteins, USSN 61/388,273 (filed September 30, 2010) relating to combinations of Cry34Ab/35Ab and Cry6Aa proteins, and USSN 61/477,447 (filed September 20, 2011) relating to combinations of Cry3Aa and Cry6Aa proteins, some preferred "triple stacks" or "multiple modes of action stacks" of the subject invention include a Cry3Ba protein combined with Cry34Ab/35Ab proteins, together with a Cry6Aa protein and/or a Cry3Aa protein. Transgenic plants, including corn, comprising a cry3Ba gene, cry34Ab/35Ab genes, and a third or fourth toxin system (e.g., cry3Aa and/or cry6Aa gene(s)) are included within the scope of the subject invention. Thus, such embodiments target the insect with at least three modes of action.

Deployment options of the subject invention include the use of Cry3Ba and Cry34Ab/35Ab proteins in corn-growing regions where *Diabrotica* spp. are problematic. Another deployment option would be to use one or both of the Cry3Ba and Cry34Ab/35Ab proteins in combination with other traits.

A person skilled in this art will appreciate that *Bt* toxins, even within a certain class such as Cry3Ba and Cry34Ab/35Ab can vary to some extent.

Genes and toxins. The term "isolated" refers to a polynucleotide in a non-naturally occurring construct, or to a protein in a purified or otherwise non-naturally occurring state. The genes and toxins useful according to the subject invention include not only the full length sequences disclosed but also fragments of these sequences, variants, mutants, and fusion proteins which retain the characteristic pesticidal activity of the toxins specifically exemplified herein. As used herein, the terms "variants" or "variations" of genes refer to nucleotide sequences which encode the same toxins or which encode equivalent toxins having pesticidal activity. As used herein, the term "equivalent toxins" refers to toxins having the same or essentially the same biological activity against the target pests as the claimed toxins. This applies to Cry3's and Cry34/35's, as well as Cry6's (if used in triple / multiple stacks) according to the subject invention. Domains/subdomains of these proteins can be swapped to make chimeric proteins. *See e.g.* U.S. Patent No. 7,309,785 and 7,524,810 regarding Cry34/35 proteins. The '785 patent also teaches truncated Cry35 proteins. Truncated toxins are also exemplified herein.

As used herein, the boundaries represent approximately 95% (Cry3Ba's and Cry34Ab's and Cry35Ab's), 78% (Cry3B's and Cry 34A's and Cry35A's), and 45% (Cry6's and Cry 34's and Cry 35's) sequence identity, per "Revision of the Nomenclature for the Bacillus thuringiensis Pesticidal Crystal Proteins," N. Crickmore, D.R. Zeigler, J. Feitelson, E. Schnepf, J. Van Rie, D. Lereclus, J. Baum, and D.H. Dean. Microbiology and Molecular Biology Reviews (1998) Vol 62: 807-813. The same applies to Cry3A's and/or Cry6's if used in triple / multiple stacks, for example, according to the subject invention.

It should be apparent to a person skilled in this art that genes encoding active toxins can be identified and obtained through several means. The specific genes or gene portions exemplified herein may be obtained from the isolates deposited at a culture depository. These genes, or portions or variants thereof, may also be constructed synthetically, for example, by use of a gene synthesizer. Variations of genes may be readily constructed using standard techniques for making point mutations. Also, fragments of these genes can be made using commercially available exonucleases or endonucleases according to standard procedures. For example, enzymes such as Bal31 or site-directed mutagenesis can be used to systematically cut off nucleotides from the ends of these genes. Genes that encode active fragments may also be obtained using a variety of restriction enzymes. Proteases may be used to directly obtain active fragments of these protein toxins.

Fragments which retain the pesticidal activity of the exemplified toxins would be within the scope of the subject invention. Also, because of the redundancy of the genetic code, a variety of different DNA sequences can encode the amino acid sequences disclosed herein. It is well within the skill of a person trained in the art to create these alternative DNA sequences encoding the same, or essentially the same, toxins. These variant DNA sequences are within the scope of the subject invention. As used herein, reference to "essentially the same" sequence refers to sequences which have amino acid substitutions, deletions, additions, or insertions which do not materially affect pesticidal activity. Fragments of genes encoding proteins that retain pesticidal activity are also included in this definition.

A further method for identifying the genes encoding the toxins and gene portions useful according to the subject invention is through the use of oligonucleotide probes. These probes are detectable nucleotide sequences. These sequences may be detectable by virtue of an appropriate label or may be made inherently fluorescent as described in International Application No. WO93/16094. As is well known in the art, if the probe molecule and nucleic acid sample hybridize by forming a strong bond between the two molecules, it can be reasonably assumed that the probe and sample have substantial homology. Preferably, hybridization is conducted under stringent conditions by techniques well-known in the art, as described, for example, in Keller, G. H., M. M. Manak (1987) DNA Probes, Stockton Press, New York, N.Y., pp. 169-170. Some examples of salt concentrations and temperature combinations are as follows (in order of increasing stringency): 2X SSPE or SSC at room temperature; 1X SSPE or SSC at 42° C; 0.1X SSPE or SSC at 42° C; 0.1X SSPE or SSC at 65° C. Detection of the probe provides a means for determining in a known manner whether hybridization has occurred. Such a probe analysis provides a rapid method for identifying toxin-encoding genes of the subject invention. The nucleotide segments which are used as probes according to the invention can be synthesized using a DNA synthesizer and standard procedures. These nucleotide sequences can also be used as PCR primers to amplify genes of the subject invention.

Variant toxins. Certain toxins of the subject invention have been specifically exemplified herein. Since these toxins are merely exemplary of the toxins of the subject invention, it should be readily apparent that the subject invention comprises variant or equivalent toxins (and nucleotide sequences coding for equivalent toxins) having the same or similar pesticidal activity of the exemplified toxin. Equivalent toxins will have amino acid homology with an exemplified toxin. This amino acid identity will typically be greater than 75%, or preferably greater than 85%, preferably greater than 90%, preferably greater than 95%, preferably greater than 96%, preferably greater than 97%, preferably greater than 98%, or preferably greater than 99% in some embodiments. The amino acid identity will typically be highest in critical regions of the toxin which account for biological activity or are involved in the determination of three-dimensional configuration which ultimately is responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Table 1 provides a listing of examples of amino acids belonging to each class.

**Table 1. Classes of amino acids with examples of amino acids belonging to each class**

| **Class of Amino Acid** | **Examples of Amino Acids** |
|---|---|
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

In some instances, non-conservative substitutions can also be made. The critical factor is that these substitutions must not significantly detract from the biological activity of the toxin.

Recombinant hosts. The genes encoding the toxins of the subject invention can be introduced into a wide variety of microbial or plant hosts. Expression of the toxin gene results, directly or indirectly, in the intracellular production and maintenance of the pesticide. Conjugal transfer and recombinant transfer can be used to create a *Bt* strain that expresses both toxins of the subject invention. Other host organisms may also be transformed with one or both of the toxin genes then used to accomplish the synergistic effect. With suitable microbial hosts, *e.g., Pseudomonas,* the microbes can be applied to the situs of the pest, where they will proliferate and be ingested. The result is control of the pest. Alternatively, the microbe hosting the toxin gene can be treated under conditions that prolong the activity of the toxin and stabilize the cell. The treated cell, which retains the toxic activity, then can be applied to the environment of the target pest. Non-regenerable / non-totipotent plant cells from a plant of the subject invention (comprising at least one of the subject IRM genes) are included within the subject invention.

Plant transformation. A preferred embodiment of the subject invention is the transformation of plants with genes encoding the subject insecticidal protein or its variants. The transformed plants are resistant to attack by an insect target pest by virtue of the presence of controlling amounts of the subject insecticidal protein or its variants in the cells of the transformed plant. By incorporating genetic material that encodes the insecticidal properties of the *B.t.* insecticidal toxins into the genome of a plant eaten by a particular insect pest, the adult or larvae would die after consuming the food plant. Numerous members of the monocotyledonous and dicotyledonous classifications have been transformed. Transgenic agronomic crops as well as fruits and vegetables are of commercial interest. Such crops include, but are not limited to, maize, rice, soybeans, canola, sunflower, alfalfa, sorghum, wheat, cotton, peanuts, tomatoes, potatoes, and the like. Several techniques exist for introducing foreign genetic material into plant cells, and for obtaining plants that stably maintain and express the introduced gene. Such techniques include acceleration of genetic material coated onto microparticles directly into cells (U.S. Patent No. 4,945,050 and U.S. Patent No. 5,141,131). Plants may be transformed using *Agrobacterium* technology, see U.S. Patent No. 5,177,010, U.S. Patent No. 5,104,310, European Patent Application No. 0131624B1, European Patent Application No. 120516, European Patent Application No. 159418B1, European Patent Application No. 176112, U.S. Patent No. 5,149,645, U.S. Patent No. 5,469,976, U.S. Patent No. 5,464,763, U.S. Patent No. 4,940,838, U.S. Patent No. 4,693,976, European Patent Application No. 116718, European Patent Application No. 290799, European Patent Application No. 320500, European Patent Application No. 604662, European Patent Application No. 627752, European Patent Application No. 0267159, European Patent Application No. 0292435, U.S. Patent No. 5,231,019, U.S. Patent No. 5,463,174, U.S. Patent No. 4,762,785, U.S. Patent No. 5,004,863, and U.S. Patent No. 5,159,135. Other transformation technology includes WHISKERS™ technology, see U.S. Patent No. 5,302,523 and U.S. Patent No. 5,464,765. Electroporation technology has also been used to transform plants, see WO 87/06614, U.S. Patent No. 5,472,869, U.S. Patent No. 5,384,253, WO 9209696, and WO 9321335. In addition to numerous technologies for transforming plants, the type of tissue which is contacted with the foreign genes may vary as well. Such tissue would include but would not be limited to embryogenic tissue, callus tissue types I and II, hypocotyl, meristem, and the like. Almost all plant tissues may be transformed during dedifferentiation using appropriate techniques within the skill of an artisan.

Genes encoding any of the subject toxins can be inserted into plant cells using a variety of techniques which are well known in the art as disclosed above. For example, a large number of cloning vectors comprising a marker that permits selection of the transformed microbial cells and a replication system functional in *Escherichia coli* are available for preparation and modification of foreign genes for insertion into higher plants. Such manipulations may include, for example, the insertion of mutations, truncations, additions, or substitutions as desired for the intended use. The vectors comprise, for example, pBR322, pUC series, M13mp series, pACYC184, *etc.* Accordingly, the sequence encoding the Cry protein or variants can be inserted into the vector at a suitable restriction site. The resulting plasmid is used for transformation of cells of *E. coli,* the cells of which are cultivated in a suitable nutrient medium, then harvested and lysed so that workable quantities of the plasmid are recovered. Sequence analysis, restriction fragment analysis, electrophoresis, and other biochemical-molecular biological methods are generally carried out as methods of analysis. After each manipulation, the DNA sequence used can be cleaved and joined to the next DNA sequence. Each manipulated DNA sequence can be cloned in the same or other plasmids.

The use of T-DNA-containing vectors for the transformation of plant cells has been intensively researched and sufficiently described in EP 120516; Lee and Gelvin (2008), Fraley *et al.* (1986), and An *et al.* (1985), and is well established in the field.

Once the inserted DNA has been integrated into the plant genome, it is relatively stable throughout subsequent generations. The vector used to transform the plant cell normally contains a selectable marker gene encoding a protein that confers on the transformed plant cells resistance to a herbicide or an antibiotic, such as bialaphos, kanamycin, G418, bleomycin, or hygromycin, *inter alia.* The individually employed selectable marker gene should accordingly permit the selection of transformed cells while the growth of cells that do not contain the inserted DNA is suppressed by the selective compound.

A large number of techniques are available for inserting DNA into a host plant cell. Those techniques include transformation with T-DNA delivered by *Agrobacterium tumefaciens* or *Agrobacterium rhizogenes* as the transformation agent. Additionally, fusion of plant protoplasts with liposomes containing the DNA to be delivered, direct injection of the DNA, biolistics transformation (microparticle bombardment), or electroporation, as well as other possible methods, may be employed.

In a preferred embodiment of the subject invention, plants will be transformed with genes wherein the codon usage of the protein coding region has been optimized for plants. See, for example, US Patent No. 5380831. Also, advantageously, plants encoding a truncated toxin will be used. The truncated toxin typically will encode about 55% to about 80% of the full length toxin. Methods for creating synthetic *B.t.* genes for use in plants are known in the art (Stewart, 2007).

Regardless of transformation technique, the gene is preferably incorporated into a gene transfer vector adapted to express the *B.t* insecticidal toxin genes and variants in the plant cell by including in the vector a plant promoter. In addition to plant promoters, promoters from a variety of sources can be used efficiently in plant cells to express foreign genes. For example, one may use promoters of bacterial origin, such as the octopine synthase promoter, the nopaline synthase promoter, and the mannopine synthase promoter. Non*-Bacillus-thuringiensis* promoters can be used in some preferred embodiments. Promoters of plant virus origin may be used, for example, the 35S and 19S promoters of Cauliflower Mosaic Virus, a promoter from Cassava Vein Mosaic Virus, and the like. Plant promoters include, but are not limited to, ribulose-1,6-bisphosphate (RUBP) carboxylase small subunit (ssu), beta-conglycinin promoter, phaseolin promoter, ADH (alcohol dehydrogenase) promoter, heat-shock promoters, ADF (actin depolymerization factor) promoter, ubiquitin promoter, actin promoter, and tissue specific promoters. Promoters may also contain certain enhancer sequence elements that may improve the transcription efficiency. Typical enhancers include but are not limited to ADH1-intron 1 and ADH1-intron 6. Constitutive promoters may be used. Constitutive promoters direct continuous gene expression in nearly all cells types and at nearly all times (*e.g.,* actin, ubiquitin, CaMV 35S). Tissue specific promoters are responsible for gene expression in specific cell or tissue types, such as the leaves or seeds (*e.g..* zein, oleosin, napin, ACP (Acyl Carrier Protein) promoters), and these promoters may also be used. Promoters may also be used that are active during a certain stage of the plants' development as well as active in specific plant tissues and organs. Examples of such promoters include but are not limited to promoters that are root specific, pollen-specific, embryo specific, corn silk specific, cotton fiber specific, seed endosperm specific, phloem specific, and the like.

Under certain circumstances it may be desirable to use an inducible promoter. An inducible promoter is responsible for expression of genes in response to a specific signal, such as: physical stimulus (*e.g.* heat shock genes); light (*e.g.* RUBP carboxylase); hormone (*e.g.* glucocorticoid); antibiotic (*e.g.* tetracycline); metabolites; and stress (*e.g.* drought). Other desirable transcription and translation elements that function in plants may be used, such as 5' untranslated leader sequences, RNA transcription termination sequences and poly-adenylate addition signal sequences. Numerous plant-specific gene transfer vectors are known to the art.

Transgenic crops containing insect resistance (IR) traits are prevalent in corn and cotton plants throughout North America, and usage of these traits is expanding globally. Commercial transgenic crops combining IR and herbicide tolerance (HT) traits have been developed by multiple seed companies. These include combinations of IR traits conferred by *B.t.* insecticidal proteins and HT traits such as tolerance to Acetolactate Synthase (ALS) inhibitors such as sulfonylureas, imidazolinones, triazolopyrimidine, sulfonanilides, and the like, Glutamine Synthetase (GS) inhibitors such as bialaphos, glufosinate, and the like, 4-HydroxyPhenylPyruvate Dioxygenase (HPPD) inhibitors such as mesotrione, isoxaflutole, and the like, 5-EnolPyruvylShikimate-3-Phosphate Synthase (EPSPS) inhibitors such as glyphosate and the like, and Acetyl-Coenzyme A Carboxylase (ACCase) inhibitors such as haloxyfop, quizalofop, diclofop, and the like. Other examples are known in which transgenically provided proteins provide plant tolerance to herbicide chemical classes such as phenoxy acids herbicides and pyridyloxyacetates auxin herbicides (see WO 2007/053482 A2), or phenoxy acids herbicides and aryloxyphenoxypropionates herbicides (see WO 2005/107437 A2, A3). The ability to control multiple pest problems through IR traits is a valuable commercial product concept, and the convenience of this product concept is enhanced if insect control traits and weed control traits are combined in the same plant. Further, improved value may be obtained via single plant combinations of IR traits conferred by a *B.t.* insecticidal protein such as that of the subject invention, with one or more additional HT traits such as those mentioned above, plus one or more additional input traits (*e.g.* other insect resistance conferred by *B.t.*-derived or other insecticidal proteins, insect resistance conferred by mechanisms such as RNAi and the like, nematode resistance, disease resistance, stress tolerance, improved nitrogen utilization, and the like), or output traits (*e.g.* high oils content, healthy oil composition, nutritional improvement, and the like). Such combinations may be obtained either through conventional breeding (breeding stack) or jointly as a novel transformation event involving the simultaneous introduction of multiple genes (molecular stack). Benefits include the ability to manage insect pests and improved weed control in a crop plant that provides secondary benefits to the producer and/or the consumer. Thus, the subject invention can be used in combination with other traits to provide a complete agronomic package of improved crop quality with the ability to flexibly and cost effectively control any number of agronomic issues.

The transformed cells grow inside the plants in the usual manner. They can form germ cells and transmit the transformed trait(s) to progeny plants.

Such plants can be grown in the normal manner and crossed with plants that have the same transformed hereditary factors or other hereditary factors. The resulting hybrid individuals have the corresponding phenotypic properties.

In a preferred embodiment of the subject invention, plants will be transformed with genes wherein the codon usage has been optimized for plants. See, for example, US Patent No. 5,380,831. In addition, methods for creating synthetic *Bt* genes for use in plants are known in the art (Stewart and Burgin, 2007). One non-limiting example of a preferred transformed plant is a fertile maize plant comprising a plant expressible gene encoding a Cry3Ba protein, and further comprising a second set of plant expressible genes encoding Cry34Ab/35Ab proteins.

Transfer (or introgression) of the Cry3Ba- and Cry34Ab/35Ab-determined trait(s) into inbred maize lines can be achieved by recurrent selection breeding, for example by backcrossing. In this case, a desired recurrent parent is first crossed to a donor inbred (the non-recurrent parent) that carries the appropriate gene(s) for the Cry-determined traits. The progeny of this cross is then mated back to the recurrent parent followed by selection in the resultant progeny for the desired trait(s) to be transferred from the non-recurrent parent. After three, preferably four, more preferably five or more generations of backcrosses with the recurrent parent with selection for the desired trait(s), the progeny will be heterozygous for loci controlling the trait(s) being transferred, but will be like the recurrent parent for most or almost all other genes (see, for example, Poehlman & Sleper (1995) Breeding Field Crops, 4th Ed., 172-175; Fehr (1987) Principles of Cultivar Development, Vol. 1: Theory and Technique, 360-376).

Insect Resistance Management (IRM) Strategies. Roush *et al.,* for example, outlines two-toxin strategies, also called "pyramiding" or "stacking," for management of insecticidal transgenic crops. (The Royal Society. Phil. Trans. R. Soc. Lond. B. (1998) 353, 1777-1786).

On their website, the United States Environmental Protection Agency (epa.gov/oppbppd1/biopesticides/pips/bt_corn_refuge_2006.htm) publishes the following requirements for providing non-transgenic (*i.e., non-B.t*.) refuges (a block of non-Bt crops / corn) for use with transgenic crops producing a single Bt protein active against target pests.

"The specific structured requirements for corn borer-protected Bt (Cry1Ab or Cry IF) corn products are as follows:
Structured refuges:
   20% non-Lepidopteran Bt corn refuge in Corn Belt;
   50% non-Lepidopteran Bt refuge in Cotton Belt
Blocks
   Internal (*i.e.,* within the Bt field)
   External (*i.e.,* separate fields within ½ mile (¼ mile if possible) of
   the Bt field to maximize random mating)
In-field Strips
   Strips must be at least 4 rows wide (preferably 6 rows) to reduce
   the effects of larval movement"

In addition, the National Corn Growers Association, on their website: (ncga. com/insect-resistance-management-fact-sheet-bt-corn) also provides similar guidance regarding the refuge requirements. For example:
"Requirements of the Corn Borer IRM:
   - Plant at least 20% of your corn acres to refuge hybrids
   - In cotton producing regions, refuge must be 50%
   - Must be planted within 1/2 mile of the refuge hybrids
   - Refuge can be planted as strips within the Bt field; the refuge strips must be at least 4 rows wide
   - Refuge may be treated with conventional pesticides only if economic thresholds are reached for target insect
   - Bt-based sprayable insecticides cannot be used on the refuge corn
   - Appropriate refuge must be planted on every farm with Bt corn"

As stated by Roush *et al.* (on pages 1780 and 1784 right column, for example), stacking or pyramiding of two different proteins each effective against the target pests and with little or no cross-resistance can allow for use of a smaller refuge. Roush suggests that for a successful stack, a refuge size of less than 10% refuge, can provide comparable resistance management to about 50% refuge for a single (non-pyramided) trait. For currently available pyramided Bt corn products, the U.S. Environmental Protection Agency requires significantly less (generally 5%) structured refuge of non-Bt corn be planted than for single trait products (generally 20%).

There are various ways of providing the IRM effects of a refuge, including various geometric planting patterns in the fields (as mentioned above) and in-bag seed mixtures, as discussed further by Roush *et al.* (*supra*), and U.S. Patent No. 6,551,962.

The above percentages, or similar refuge ratios, can be used for the subject double or triple stacks or pyramids. Because the subject invention provides multiple, non-competitive modes of action against a rootworm target insect, the subject invention could provide "zero refuge", that is, a field that lacks refuge plants (because they are not required). A permit is typically required for typical *B.t.* transgenic fields of above about 10 acres. Thus, the subject invention includes a field of 10 acres or more with "zero refuge" or no Bt plants; fields of this size would previously have been required to have a significant non-Bt refuge.

Following are examples that illustrate procedures for practicing the invention. These examples should not be construed as limiting. All percentages are by weight and all solvent mixture proportions are by volume unless otherwise noted. All temperatures are in degrees Celsius.

Unless specifically indicated or implied, the terms "a", "an", and "the" signify "at least one" as used herein.

### EXAMPLES

### Example 1- Construction of expression plasmids encoding Cry34Ab1, Cry35Ab1, and Cry3Ba1 full-length toxins

Standard cloning methods were used in the construction of *Pseudomonas fluorescens* (*Pf*) expression plasmids engineered to produce full-length Cry34Ab1, Cry35Ab1, and Cry3Ba1 Cry proteins respectively. Restriction endonucleases from New England BioLabs (NEB; Ipswich, MA) were used for DNA digestion and T4 DNA Ligase from Invitrogen was used for DNA ligation. Plasmid preparations were performed using the Plasmid Midi Kit (Qiagen), following the instructions of the supplier. DNA fragments were purified using the Millipore Ultrafree®-DA cartridge (Billerica, MA) after agarose Tris-acetate gel electrophoresis. The basic cloning strategy entailed subcloning the coding sequences (CDS) of a full-length Cry34Ab1 and Cry35Ab1 proteins into pMYC1803 at *SpeI* and *XhoI* (or *XbaI*) restriction sites, and the CDS of a full-length Cry3Ba1 protein into pMYC1050 at *KpnI* and *XbaI* restriction sites, respectively, whereby they were placed under the expression control of the *Ptac* promoter and the rrnBT1T2 terminator from plasmid pKK223-3 (PL Pharmacia, Milwaukee, WI), respectively. pMYC1803 is a medium copy plasmid with the RSF1010 origin of replication, a *tetracycline resistance* gene, and a ribosome binding site preceding the restriction enzyme recognition sites into which DNA fragments containing protein coding regions may be introduced (U.S. Patent Application No. 2008/0193974). The expression plasmid was transformed by electroporation into a *P. fluorescens* strain MB214, recovered in SOC-Soy hydrolysate medium, and plated on Lysogeny broth (LB) medium containing 20 µg/ml tetracycline. Details of the microbiological manipulations are available U.S. Patent Application No. 2006/0008877, U.S. Patent Application No. 2008/0193974, and U.S. Patent Application No. 2008/0058262.

Colonies were screened by restriction digestion of miniprep plasmid DNA. Plasmid DNA of selected clones containing inserts was sequenced by contract with a commercial sequencing vendor such as MWG Biotech (Huntsville, AL). Sequence data were assembled and analyzed using the Sequencher™ software (Gene Codes Corp., Ann Arbor, MI).

### Example 2 - Growth and expression

Growth and expression analysis in shake flasks production of Cry34Ab1, Cry35Ab1, and Cry3Ba1 toxins for characterization including Bt receptor binding and insect bioassay was accomplished by shake-flask-grown *P. fluorescens* strains harboring expression constructs (*e.g.* clone pMYC2593 for Cry34Ab1, pMYC3122 for Cry35Ab1, and pMYC1177 for Cry3Ba1). Seed cultures grown in LB medium supplemented with 20 µg/ml tetracycline were used to inoculate 200 mL of the same medium with 20 µg/ml tetracycline. Expression of Cry34Ab1, and Cry3Ba1 toxins via the *Ptac* promoter was induced by addition of isopropyl-β-D-1-thiogalactopyranoside (IPTG) after an initial incubation of 24 hours at 30°C with shaking. Cultures were sampled at the time of induction and at various times post-induction. Cell density was measured by optical density at 600 nm (OD₆₀₀).

### Example 3 - Cell fractionation and SDS-PAGE analysis ofshake flask samples

At each sampling time, the cell density of samples was adjusted to OD₆₀₀ = 20 and 1 mL aliquots are centrifuged at 14,000 x g for five minutes. The cell pellets were frozen at -80°C. Soluble and insoluble fractions from frozen shake flask cell pellet samples were generated using EasyLyse™ Bacterial Protein Extraction Solution (EPICENTRE® Biotechnologies, Madison, WI). Each cell pellet was resuspended in 1 mL EasyLyse™ solution and further diluted 1:4 in lysis buffer and incubated with shaking at room temperature for 30 minutes. The lysate was centrifuged at 14,000 rpm for 20 minutes at 4°C and the supernatant was recovered as the soluble fraction. The pellet (insoluble fraction) was then resuspended in an equal volume of phosphate buffered saline (PBS; 11.9 mM Na₂HPO₄, 137 mM NaCl, 2.7 mM KCl, pH7.4). Samples were mixed 1:1 with 2X Laemmli sample buffer containing β-mercaptoethanol and boiled for 5 minutes prior to loading onto NuPAGE Novex 4-20% Bis-Tris gels (Invitrogen, Carlsbad, CA). Electrophoresis was performed in the recommended XT MOPS buffer. Gels were stained with the SimplyBlue™ Safe Stain according to the manufacturer's (Invitrogen) protocol and imaged using the Typhoon imaging system (GE Healthcare Life Sciences, Pittsburgh, PA).

### Example 4 - Inclusion body preparation

Cry protein inclusion body (IB) preparations were performed on cells from P. *fluorescens* fermentations that produced insoluble *B.t.* insecticidal protein, as demonstrated by SDS-PAGE and MALDI-MS (Matrix Assisted Laser Desorption/Ionization Mass Spectrometry). *P. fluorescens* fermentation pellets are thawed in a 37°C water bath. The cells were resuspended to 25% w/v in lysis buffer [50 mM Tris, pH 7.5, 200 mM NaCl, 20 mM EDTA disodium salt (Ethylenediaminetetraacetic acid), 1% Triton X-100, and 5 mM Dithiothreitol (DTT)] and 5 mL/L of bacterial protease inhibitor cocktail (P8465 Sigma-Aldrich, St. Louis, MO) was added just prior to use. The cells were suspended using a homogenizer at lowest setting (Tissue Tearor, BioSpec Products, Inc., Bartlesville, OK). Lysozyme (25 mg of Sigma L7651, from chicken egg white) was added to the cell suspension by mixing with a metal spatula, and the suspension was incubated at room temperature for one hour. The suspension was cooled on ice for 15 minutes, then sonicated using a Branson Sonifier 250 (two 1- minute sessions, at 50% duty cycle, 30% output). Cell lysis was checked by microscopy. An additional 25 mg of lysozyme was added if necessary, and the incubation and sonication were repeated. When cell lysis was confirmed via microscopy, the lysate was centrifuged at 11,500 x g for 25 minutes (4°C) to form the IB pellet, and the supernatant was discarded. The IB pellet was resuspended with 100 mL lysis buffer, homogenized with the hand-held mixer and centrifuged as above. The IB pellet was repeatedly washed by resuspension (in 50 mL lysis buffer), homogenization, sonication, and centrifugation until the supernatant became colorless and the IB pellet became firm and off-white in color. For the final wash, the IB pellet was resuspended in sterile-filtered (0.22 µm) distilled water containing 2 mM EDTA, and centrifuged. The final pellet was resuspended in sterile-filtered distilled water containing 2 mM EDTA, and stored in 1 mL aliquots at -80°C.

### Example 5 - SDS PAGE analysis and quantification

SDS-PAGE analysis and quantification of protein in IB preparations were done by thawing a 1 mL aliquot of IB pellet and diluting 1:20 with sterile-filtered distilled water. The diluted sample was then boiled with 4X reducing sample buffer [250 mM Tris, pH6.8, 40% glycerol (v/v), 0.4% Bromophenol Blue (w/v), 8% SDS (w/v) and 8% β-Mercapto-ethanol (v/v)] and loaded onto a Novex® 4-20% Tris-Glycine, 12+2 well gel (Invitrogen) run with 1X Tris/Glycine/SDS buffer (Invitrogen). The gel was run for approximately 60 min at 200 volts then stained and destained by following the SimplyBlue™ Safe Stain (Invitrogen) procedures. Quantification of target bands was done by comparing densitometric values for the bands against Bovine Serum Albumin (BSA) samples run on the same gel to generate a standard curve using the Bio-Rad Quantity One® software.

### Example 6 - Solubilization of inclusion bodies

Ten mL of inclusion body suspensions from *P. fluoresces* clones MR1253, MR1636, and MR816 (containing 50-70 mg/mL of Cry34Ab1, and Cry3Ba1 proteins respectively) were centrifuged at the highest setting of an Eppendorf model 5415C microfuge (approximately 14,000 x g) to pellet the inclusions. The storage buffer supernatant was removed and replaced with 25 mL of 100 mM sodium acetate buffer, pH 3.0, for both Cry34Ab1 and and 100 mM sodium carbonate buffer, pH11, for Cry3Ba1, in a 50 mL conical tube, respectively. Inclusions were resuspended using a pipette and vortexed to mix thoroughly. The tubes were placed on a gently rocking platform at 4°C overnight to extract full-length Cry34Ab1, and Cry3Ba1 proteins. The extracts were centrifuged at 30,000 x g for 30 min at 4°C, and the resulting supernatants (containing solubilized full-length Cry proteins) were saved.

### Example 7 - Truncation of full-length protoxins

Full-length Cry35Ab1 and Cry3Ba1 were truncated or digested with chymotrypsin or trypsin to produce chymotrypsin or trypsin core fragments that are an active form of the proteins. Specially, the solubilized full-length Cry35Ab1 was incubated with chymotrypsin (bovine pancreas) (Sigma, St. MO) (at 50:1 = Cry protein: enzyme, w/w) in the 100 mM sodium acetate buffer, pH 3.0 (Example 6), at 4°C with gentle shaking for 2-3 days, while full-length Cry3Ba1 was incubated with trypsin (bovine pancreas) (Sigma, St. MO) (at 20:1 = Cry protein: enzyme, w/w) in the 100 mM sodium carbonate buffer, pH11 (Example 6), at room temperature for 1-3 hours. Complete proteolytic preocessing was confirmed by SDS-PAGE analysis. The molecular mass of the full-length Cry35Ab1 and Cry3Ba1 was approximately equal to 44 and approximately equal to 73 kDa, and their chymotrypsin or trypsin core was approximately equal to 40 and approximately equal to 55 kDa, respectively. The amino acid sequences of full-length and chymotrypsin core of Cry35Ab1 are provided as SEQ ID 1 and SEQ ID 2, and the amino acid sequences of full-length and trypsin core of Cry3Ba1 are provide as SEQ ID 3 and SEQ ID 4. Either chymotrypsin or trypsin core is not available for Cry34Ab1, and thus the full-length Cry34Ab1 was used for binding assays. The amino acid sequence of the full-length Cry34Ab1 is provided as SEQ ID 5.

### Example 8 - Purification of truncated toxins

The chymotrypsinized Cry35Ab1 and trypsinized Cry3Ba1 core fragments were purified. Specifically, the digestion reactions were centrifuged at 30,000 x g for 30 min at 4°C to remove lipids, and the resulting supernatant were concentrated 5-fold using an Amicon Ultra-15 regenerated cellulose centrifugal filter device (10,000 Molecular Weight Cutoff; Millipore). The sample buffers were then changed to 20 mM sodium acetate buffer, pH 3.5, for both Cry34Ab1 and Cry35Ab1,and to 10 mM CAPS [3-(cyclohexamino)1-propanesulfonic acid], pH 10.5, for Cry3Ba1, using disposable PD-10 columns (GE Healthcare, Piscataway, NJ) or dialysis. The final volumes were adjusted to 15 ml using the corresponding buffer for purification using ATKA Explorer liquid chromatography system (Amersham Biosciences). For buffer A was 20 mM sodium acetate buffer, pH 3.5, and buffer B was buffer A + 1 M NaCl, pH 3.5. A HiTrap SP (5 ml) column (GE) was used. After the column was fully equilibrated using the buffer A, the Cry35Ab1 solution was injected into the column at a flow rate of 5 ml/min. Elution was performed using gradient 0-100% of buffer B at 5 ml/min with 1 ml/fraction. For Cry3Ba1, buffer A was 10 mM CAPS buffer, pH 10.5, and buffer B was 10 mM CAPS buffer, pH 10.5 + 1 M NaCl. A Capto Q, 5 ml (5 ml) column (GE) was used and all other procedures were similar to that for Cry35Ab1. After SDS-PAGE analysis of the selected fractions to further select fractions containing the best quality target protein, pooled those fractions. The buffer was changed for the purified Cry35Ab1 chymotrypsin core with 20 mM Bist-Tris, pH 6.0, as described above. For the purified Cry3Ba1 trypsin core, the salt was removed using disposable PD-10 columns (GE Healthcare, Piscataway, NJ) or dialysis. The samples were saved at 4°C for later binding assays after being quantified using SDS-PAGE and the Typhoon imaging system (GE) analysis with BSA as a standard.

### Example 9 - BBMV preparation

Brush border membrane vesicle (BBMV) preparations of insects have been widely used for Cry toxin receptor binding assays. The BBMV preparations used in this invention were prepared from isolated midguts of third instars of the western corn rootworm (*Diabrotica virgifera virgifera* LeConte) using the method described by Wolferberger et al. (1987). Leucine aminopeptidase was used as a marker of membrane proteins in the preparation and Leucine aminopeptidase activities of crude homogenate and BBMV preparations were determined as previously described (Li et al. 2004a). Protein concentration of the BBMV preparations were measured using the Bradford method (1976).

### Example 10 - ¹²⁵I Labeling

Purified full-length Cry34Ab1, chymotrypsinized and trypsinized Cry3Ba1 were labeled using ¹²⁵I for saturation and homologous competition binding assays. To ensure the radio-labeling does not abolish the biological activity of the Cry toxins, cold iodination was conducted using NaI by following the instructions of Pierce® Iodination Beads (Pierce Biotechnology, Thermo Scientific, Rockford IL). Bioassay results indicated that iodinated Cry35Ab1 chymotrypsin core remained active against the larvae of the western corn rootworm, but iodination inactivated Cry34Ab1. The specific binding of radiolabeled ¹²⁵I-Cry34Ab1 to the insect BBMV was not able to be detected, and thus requiring another labeling method to assess membrane receptor binding of Cry34Ab1. Since trypsinized Cry3Ba1 had limited activity against western corn rootworm, and thus the bioassay with the corn rootworm using cold iodinated Cry3Ba1 trypsin core was thought difficult to assess the activity change. In addition, the specific binding of ¹²⁵I-Cry3Ba1 to the BBMV was detected even though the level was low. Cold-iodination of Cry3Ba1 and its toxicity assay were ignored. Radiolabeled ¹²⁵I-Cry35Ab1 and ¹²⁵I-Cry3Ba1 were obtained through iodination with Pierce® Iodination Beads (Pierce) and Na¹²⁵I. Zeba™ Desalt Spin Columns (Pierce) were used to remove unincorporated or free Na¹²⁵I from the iodinated protein. The specific radioactivities of the iodinated Cry proteins ranged from 1-5 uCi/ug. Multiple batches of labeling and binding assays were conducted.

### Example 11 - Saturation binding assays

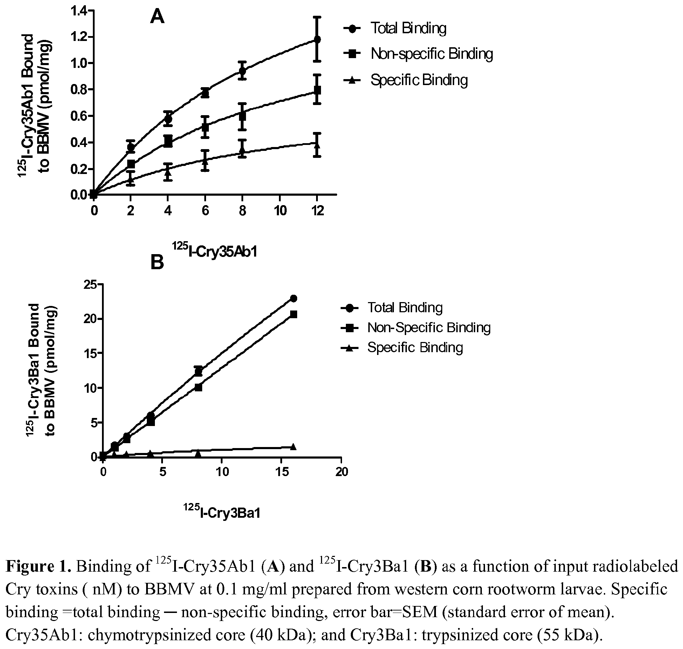

Specific or saturation binding assays were performed using ¹²⁵I-labeled Cry toxins as described previously (Li et al. 2004b). To determine specific binding and estimate the binding affinity (disassociation constant, Kd) and binding site concentration (Bmax) of Cry35Ab1 and Cry3Ba1 to the insect BBMV, a series of increasing concentrations of either ¹²⁵I-Cry35Ab1 or ¹²⁵I-Cry3Ba1 were incubated with a given concentration (0.1 mg/ml) of the insect BBMV, respectively, in 150 ul of 20 mM Bis-Tris, pH 6.0, 150 mM KCl, supplemented with 0.1% BSA at room temperature for 1 hour with gentle shaking. Toxin bound to BBMV was separated from free toxin in the suspension by centrifugation at 20,000 x g at room temperature for 8 min. The pellet was washed twice with (ice-cold) 900 ul of the same buffer containing 0.1% BSA. The radioactivity remaining in the pellet was measured with a COBRAII Auto-Gamma counter (Packard, a Canberra company) and considered total binding. Another series of binding reactions were setup as side by side, and a 500-1,000-fold excess of unlabeled corresponding toxin was included in each of the binding reactions to fully occupy all specific binding sites on the BBMV, which was used to determine non-specific binding. Specific binding was estimated by subtracting the non-specific binding from the total binding. The Kd and Bmax values of these toxins were estimated using the specific binding against the concentrations of the labeled toxin used by running GraphPad Prism 5.01 (GraphPad Software, San Diego, CA). The charts were made using either Microsoft Excel or GraphPad Prism software. The experiments were replicated at least four times and the result plotted in the graphs of Figures 1A (binding of ¹²⁵I-Cry35Ab1 to BBMV) and Figure 1B (binding of ¹²⁵I-Cry3Ba1 to BBMV). These binding experiments demonstrated that both ¹²⁵I-Cry35Ab1 and ¹²⁵I-Cry3Ba1 were able to specifically bind to the BBMV (Figure 1A and 1B). ¹²⁵I-Cry35Ab1 and ¹²⁵I-Cry3Ba1 had a binding affinity Kd=11.66±11.44, 7.35±3.81 (nM), respectively, and a binding site concentration Bmax=0.78±0.46, 0.55±0.13 (pmol/mg BBMV), respectively.

The specific binding of ¹²⁵I-Cry35Ab1 was carried out at the presence of unlabeled Cry34Ab1 (1:50=¹²⁵I-Cry35Ab1:Cry34Ab1, molar ratio). The binding parameters (Kd and Bmax) were not obtained because the specific binding of ¹²⁵I-Cry35Ab1 was not saturated (Fig.2). However, the specific binding of ¹²⁵I-Cry35Ab1 accounted for approximately 90% of the total binding at the presence of unlabeled Cry34Ab1.

### Example 12 - Competition binding assays

Competition binding assays were conducted to determine if Cry34Ab1 and Cry35Ab1 separately, plus their mixture as a binary toxin, share a same set of binding sites with Cry3Ba1. For homologous competition binding assays of Cry3Ba1, increasing amounts (0-2,500 nM) of unlabeled Cry3Ba1 were first mixed with 5 nM ¹²⁵I-Cry3Ba1, and then incubated the insect BBMV at with 0.1 mg/ml at room temperature for 1 hour, respectively, to allow them compete for the putative receptor(s) on the BBMV. Similarly, Cry35Ab1 homologous competition was completed with 5 nM ¹²⁵I-Cry35Ab1 at the absence or presence of unlabeled Cry34Ab1 (1:50 = ¹²⁵I-Cry35Ab1:Cry34Ab1, molar ratio), and with the BBMV at 0.03 mg/ml, respectively. The percentages of bound ¹²⁵I-Cry3Ba1 or ¹²⁵I-Cry35Ab1 to the BBMV were determined for each of the reactions as compared to the initial total (or specific) binding at absence of unlabeled competitor.

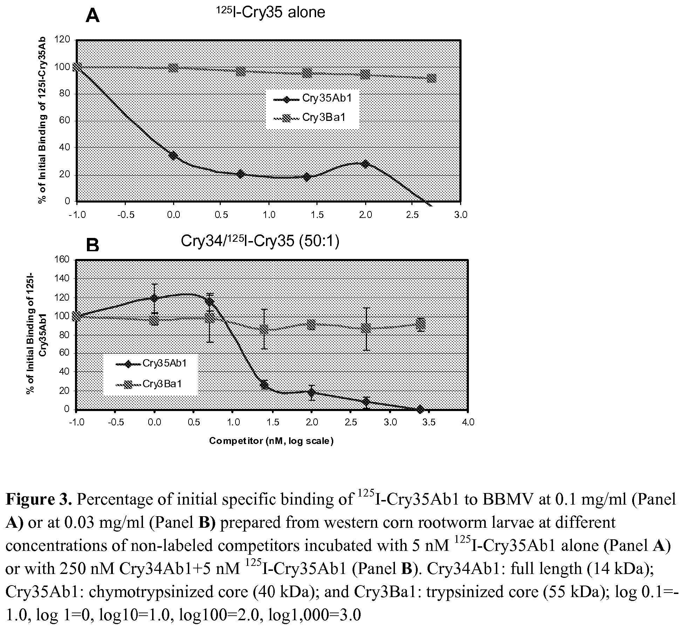

Heterologous competition binding assays between ¹²⁵I-Cry35Ab1 and unlabeled Cry3Ba1 were conducted at absence or presence of unlabeled Cry34Ab1 to identify if they share a same set of binding site(s). This was achieved by increasing the amount of unlabeled Cry3Ba1 as a competitor to compete for the binding with ¹²⁵I-Cry35Ab1 alone or ¹²⁵I-Cry35Ab1+Cry34Ab1 (1:50=¹²⁵I-Cry35Ab1:Cry34Ab1, molar ratio). Similarly, reciprocal heterologous competition binding assays were also conducted, which was achieved by increasing the amount of unlabeled Cry35Ab1 and Cry34Ab1 separately, or the Cry35Ab1+Cry34Ab1 (1:50=Cry35Ab1:Cry34Ab1, molar ratio) mixture, as one or two competitors included in the reactions to compete for the binding with the labeled Cry3Ba1, respectively. The experiments were replicated at least three times and the result plotted in the graphs of Figure 3A (percent binding of ¹²⁵I-Cry35Ab alone) and Figure 3B (percent binding of ¹²⁵I-Cry35Ab1 in the presence of Cry34Ab1).

The experimental results demonstrated that Cry35Ab1 was able to compete off the specific binding of ¹²⁵I-Cry35Ab1 regardless of absence (Fig. 3A) or presence (Fig 3B) of Cry34Ab1. However, Cry3Ba1 was unable to compete off the specific binding of ¹²⁵I-Cry35Ab1 at either absence or presence of Cry34Ab1. In reciprocal competition binding assays, Cry3Ba was also able to displace itself over 20% of the total binding, which reflects it completely competed off its specific binding because the specific binding accounts only a small fraction (see Fig.1B). However, either Cry34Ab1, or Cry35Ab1 alone, or the mixture of Cry35Ab1+Cry34Ab1 (1:10) was not able to displace ¹²⁵I-Cry3Ba1. These data indicated that Cry35Ab1 alone or mixture of Cry35Ab1+Cry34Ab1 does not share a receptor binding site with Cry3Ba1.

### References:

Bradford, M.M. 1976. A rapid and sensitive method for the quantitation of microgram quantities of protein utilizing the principle of protein-dye binding, Anal. Biochem. 72, 248-254.
Li, H., Oppert, B., Higgins, R.A., Huang, F., Zhu, K.Y., Buschman, L.L., 2004a. Comparative analysis of proteinase activities of Bacillus thuringiensis-resistant and -susceptible Ostrinia nubilalis (Lepidoptera: Crambidae). Insect Biochem. Mol. Biol. 34, 753-762.
Li, H., Oppert, B., Gonzalez-Cabrera, J., Ferré, J., Higgins, R.A., Buschman, L.L. and Zhu, K.Y. and Huang, F. 2004b. Binding analysis of Cry1Ab and Cry1Ac with membrane vesicles from Bacillus thuringiensis-resistant and -susceptible Ostrinia nubilalis (Lepidoptera: Crambidae). Biochem. Biophys. Res. Commun. 323, 52-57.
Wolfersberger, M.G., Luthy, P., Maurer, A., Parenti, P., Sacchi, F., Giordana, B., Hanozet, G.M., 1987. Preparation and partial characterization of amino acid transporting brush border membranevesicles from the larval midgut of the cabbage butterfly (Pieris brassicae). Comp. Biochem. Physiol. 86A, 301-308.
US Patent Application No. 20080193974. 2008. BACTERIAL LEADER SEQUENCES FOR INCREASED EXPRESSION
US Patent Application No. 20060008877, 2006. Expression systems with sec-system secretion.
US Patent Application No. 20080058262, 2008. rPA optimization.

## Claims

1. A transgenic plant that expresses a Cry34Ab insecticidal protein, a Cry35Ab insecticidal protein, and a Cry3Ba insecticidal protein.

2. The transgenic plant according to claim 1, wherein said plant further expresses a fourth insecticidal protein selected from the group consisting of Cry3Aa and Cry6Aa.

3. A seed of the transgenic plant according to claim 1 or 2, wherein said seed comprises DNA encoding the Cry34Ab insecticidal protein, the Cry35Ab insecticidal protein, and the Cry3Ba insecticidal protein.

4. A plurality of transgenic plants according to claim 1 or 2.

5. The plant according to claim 1 or 2, wherein said plant is a maize plant.

6. A plant cell of the plant according to claim 1 or 2, wherein said Cry35Ab insecticidal protein is at least 95% identical with a sequence selected from the group consisting of SEQ ID NO:1 and SEQ ID NO:2, said Cry3Ba insecticidal protein is at least 95% identical with a sequence selected from the group consisting of SEQ ID NO:3 and SEQ ID NO:4, and said Cry34Ab insecticidal protein is at least 95% identical with SEQ ID NO:5.

7. The transgenic plant according to claim 1 or 2, wherein said Cry35Ab insecticidal protein comprises a sequence selected from the group consisting of SEQ ID NO:1 and SEQ ID NO:2, said Cry3Ba insecticidal protein comprises a sequence selected from the group consisting of SEQ ID NO:3 and SEQ ID NO:4, and said Cry34Ab insecticidal protein comprises SEQ ID NO:5.

8. A method of producing the plant cell according to claim 6, said method comprising transforming a plant cell with DNA capable of expressing the proteins according to claim 1 or 2.

9. A method of controlling a corn rootworm insect by contacting said insect with a Cry34Ab insecticidal protein, a Cry35Ab insecticidal protein, and a Cry3Ba insecticidal protein.

10. The transgenic plant according to claim 1, wherein said Cry34Ab insecticidal protein is a Cry34Ab1 insecticidal protein, said Cry35Ab insecticidal protein is a Cry35Ab1 insecticidal protein, and said Cry3Ba insecticidal protein is a Cry3Bal insecticidal protein.

## Patentansprüche

1. Eine transgene Pflanze, die ein Cry34Ab insektizides Protein, ein Cry35Ab insektizides Protein und ein Cry3Ba insektizides Protein exprimiert.

2. Die transgene Pflanze gemäß Anspruch 1, wobei die Pflanze weiter ein viertes insektizides Protein, ausgesucht aus der Gruppe bestehend aus Cry3Aa und Cry6Aa exprimiert.

3. Ein Samen einer transgenen Pflanze gemäß Anspruch 1 oder 2, wobei genannter Samen eine DNA umfasst, die das Cry34Ab insektizide Protein, das Cry35Ab insektizide Protein und das Cry3Ba insektizide Protein kodiert.

4. Eine Vielzahl transgener Pflanzen gemäß Anspruch 1 oder 2.

5. Die Pflanze gemäß Anspruch 1 oder 2, wobei die genannte Pflanze eine Maispflanze ist.

6. Eine Pflanzenzelle einer Pflanze gemäß Anspruch 1 oder 2, wobei genanntes Cry35Ab insektizides Protein zumindest 95 % identisch ist mit einer Sequenz ausgesucht aus der Gruppe bestehend aus SEQ ID Nr. 1 und SEQ ID Nr. 2, genanntes Cry3Ba insektizides Protein zumindest 95 % identisch ist mit einer Sequenz ausgesucht aus der Gruppe bestehend aus SEQ ID Nr. 3 und SEQ ID Nr. 4, und genanntes Cry34Ab insektizides Protein zumindest 95 % identisch ist mit SEQ ID Nr. 5.

7. Die transgene Pflanze gemäß Anspruch 1 oder 2, wobei genanntes Cry35Ab insektizides Protein eine Sequenz umfasst, ausgesucht aus der Gruppe bestehend aus SEQ ID Nr. 1 und SEQ ID Nr. 2, genanntes Cry3Ba insektizides Protein eine Sequenz umfasst ausgesucht aus der Gruppe bestehend aus SEQ ID Nr. 3 und SEQ ID Nr. 4, und genanntes Cry34Ab insektizides Protein SEQ ID Nr. 5 umfasst.

8. Ein Verfahren zur Herstellung einer Pflanzenzelle gemäß Anspruch 6, wobei genanntes Verfahren die Transformierung einer Pflanzenzelle mit einer DNA umfasst, die fähig ist, die Proteine gemäß Anspruch 1 oder 2 zu exprimieren.

9. Ein Verfahren zur Kontrolle eines Maiswurzelbohrerinsekts durch In-Kontakt-Bringen des genannten Insekts mit einem Cry34Ab insektiziden Protein, einem Cry35Ab insektiziden Protein und einem Cry3Ba insektiziden Protein.

10. Die transgene Pflanze gemäß Anspruch 1, wobei genanntes Cry34Ab insektizides Protein ein Cry34Ab1 insektizides Protein ist, genanntes Cry35Ab insektizides Protein ein Cry35Ab1 insektizides Protein ist und genanntes Cry3Ba insektizides Protein ein Cry3Ba1 insektizides Protein ist.

## Revendications

1. Plante transgénique qui exprime une protéine insecticide Cry34Ab, une protéine insecticide Cry35Ab et une protéine insecticide Cry3Ba.

2. Plante transgénique selon la revendication 1, laquelle plante exprime en outre une quatrième protéine insecticide choisie dans l'ensemble constitué par Cry3Aa et Cry6Aa.

3. Graine de la plante transgénique selon la revendication 1 ou 2, laquelle graine comprend un ADN codant la protéine insecticide Cry34Ab, la protéine insecticide Cry35Ab, et la protéine insecticide Cry3Ba.

4. Pluralité de plantes transgéniques selon la revendication 1 ou 2.

5. Plante selon la revendication 1 ou 2, laquelle plante est un plan de maïs.

6. Cellule végétale de la plante selon la revendication 1 ou 2, dans laquelle ladite protéine insecticide Cry35Ab est identique à au moins 95 % à une séquence choisie dans l'ensemble constitué par la SEQ ID NO : 1 et la SEQ ID NO : 2, ladite protéine insecticide Cry3Ba est identique à au moins 95 % à une séquence choisie dans l'ensemble constitué par la SEQ ID NO : 3 et la SEQ ID NO : 4, et ladite protéine insecticide Cry34Ab est identique à au moins 95 % à la SEQ ID NO : 5.

7. Plante transgénique selon la revendication 1 ou 2, dans laquelle ladite protéine insecticide Cry35Ab comprend une séquence choisie dans l'ensemble constitué par la SEQ ID NO : 1 et la SEQ ID NO : 2, ladite protéine insecticide Cry3Ba comprend une séquence choisie dans l'ensemble constitué par la SEQ ID NO : 3 et la SEQ ID NO : 4, et ladite protéine insecticide Cry34Ab comprend la SEQ ID NO : 5.

8. Procédé pour produire la cellule végétale de la revendication 6, ledit procédé comprenant la transformation d'une cellule végétale avec un ADN capable d'exprimer les protéines selon la revendication 1 ou 2.

9. Procédé pour lutter contre l'insecte chrysomèle des racines du maïs par mise en contact dudit insecte avec une protéine insecticide Cry34Ab, une protéine insecticide Cry35Ab, et une protéine insecticide Cry3Ba.

10. Plante transgénique selon la revendication 1, dans laquelle ladite protéine insecticide Cry34Ab est une protéine insecticide Cry34Ab1, ladite protéine insecticide Cry35Ab est une protéine insecticide et ladite protéine insecticide Cry3Ba est une protéine insecticide Cry3Ba1.


### SEQUENCE LISTING

<110> DOW AGROSCIENCES
<120> Combinations of Cry34Ab1/35Ab1 and Cry3Ba1 Proteins to Prevent Development of Resistance in Corn Rootworms
<130> DAS-P0172-US-02
<160> 5
<170> PatentIn version 3.5
<210> 1
   <211> 383
   <212> PRT
   <213> Bacillus thuringiensis
<400> 1
   Met Leu Asp Thr Asn Lys Val Tyr Glu Ile Ser Asn His Ala Asn Gly
   1               5                   10                  15
   Leu Tyr Ala Ala Thr Tyr Leu Ser Leu Asp Asp Ser Gly Val Ser Leu
               20                  25                  30
   Met Asn Lys Asn Asp Asp Asp Ile Asp Asp Tyr Asn Leu Lys Trp Phe
           35                  40                  45
   Leu Phe Pro Ile Asp Asp Asp Gln Tyr Ile Ile Thr Ser Tyr Ala Ala
       50                  55                  60
   Asn Asn Cys Lys Val Trp Asn Val Asn Asn Asp Lys Ile Asn Val Ser
   65                  70                  75                  80
   Thr Tyr Ser Ser Thr Asn Ser Ile Gln Lys Trp Gln Ile Lys Ala Asn
                   85                  90                  95
   Gly Ser Ser Tyr Val Ile Gln Ser Asp Asn Gly Lys Val Leu Thr Ala
               100                 105                 110
   Gly Thr Gly Gln Ala Leu Gly Leu Ile Arg Leu Thr Asp Glu Ser Ser
           115                 120                 125
   Asn Asn Pro Asn Gln Gln Trp Asn Leu Thr Ser Val Gln Thr Ile Gln
       130                 135                 140
   Leu Pro Gln Lys Pro Ile Ile Asp Thr Lys Leu Lys Asp Tyr Pro Lys
   145                 150                 155                 160
   Tyr Ser Pro Thr Gly Asn Ile Asp Asn Gly Thr Ser Pro Gln Leu Met
                   165                 170                 175
   Gly Trp Thr Leu Val Pro Cys Ile Met Val Asn Asp Pro Asn Ile Asp
               180                 185                 190
   Lys Asn Thr Gln Ile Lys Thr Thr Pro Tyr Tyr Ile Leu Lys Lys Tyr
           195                 200                 205
   Gln Tyr Trp Gln Arg Ala Val Gly Ser Asn Val Ala Leu Arg Pro His
       210                 215                 220
   Glu Lys Lys Ser Tyr Thr Tyr Glu Trp Gly Thr Glu Ile Asp Gln Lys
   225                 230                 235                 240
   Thr Thr Ile Ile Asn Thr Leu Gly Phe Gln Ile Asn Ile Asp Ser Gly
                   245                 250                 255
   Met Lys Phe Asp Ile Pro Glu Val Gly Gly Gly Thr Asp Glu Ile Lys
               260                 265                 270
   Thr Gln Leu Asn Glu Glu Leu Lys Ile Glu Tyr Ser His Glu Thr Lys
           275                 280                 285
   Ile Met Glu Lys Tyr Gln Glu Gln Ser Glu Ile Asp Asn Pro Thr Asp
       290                 295                 300
   Gln Ser Met Asn Ser Ile Gly Phe Leu Thr Ile Thr Ser Leu Glu Leu
   305                 310                 315                 320
   Tyr Arg Tyr Asn Gly Ser Glu Ile Arg Ile Met Gln Ile Gln Thr Ser
                   325                 330                 335
   Asp Asn Asp Thr Tyr Asn Val Thr Ser Tyr Pro Asn His Gln Gln Ala
               340                 345                 350
   Leu Leu Leu Leu Thr Asn His Ser Tyr Glu Glu Val Glu Glu Ile Thr
           355                 360                 365
   Asn Ile Pro Lys Ser Thr Leu Lys Lys Leu Lys Lys Tyr Tyr Phe
       370                 375                 380
<210> 2
   <211> 354
   <212> PRT
   <213> Bacillus thuringiensis
<400> 2
   Met Leu Asp Thr Asn Lys Val Tyr Glu Ile Ser Asn His Ala Asn Gly
   1               5                   10                  15
   Leu Tyr Ala Ala Thr Tyr Leu Ser Leu Asp Asp Ser Gly Val Ser Leu
               20                  25                  30
   Met Asn Lys Asn Asp Asp Asp Ile Asp Asp Tyr Asn Leu Lys Trp Phe
           35                  40                  45
   Leu Phe Pro Ile Asp Asp Asp Gln Tyr Ile Ile Thr Ser Tyr Ala Ala
       50                  55                  60
   Asn Asn Cys Lys Val Trp Asn Val Asn Asn Asp Lys Ile Asn Val Ser
   65                  70                  75                  80
   Thr Tyr Ser Ser Thr Asn Ser Ile Gln Lys Trp Gln Ile Lys Ala Asn
                   85                  90                  95
   Gly Ser Ser Tyr Val Ile Gln Ser Asp Asn Gly Lys Val Leu Thr Ala
               100                 105                 110
   Gly Thr Gly Gln Ala Leu Gly Leu Ile Arg Leu Thr Asp Glu Ser Ser
           115                 120                 125
   Asn Asn Pro Asn Gln Gln Trp Asn Leu Thr Ser Val Gln Thr Ile Gln
       130                 135                 140
   Leu Pro Gln Lys Pro Ile Ile Asp Thr Lys Leu Lys Asp Tyr Pro Lys
   145                 150                 155                 160
   Tyr Ser Pro Thr Gly Asn Ile Asp Asn Gly Thr Ser Pro Gln Leu Met
                   165                 170                 175
   Gly Trp Thr Leu Val Pro Cys Ile Met Val Asn Asp Pro Asn Ile Asp
               180                 185                 190
   Lys Asn Thr Gln Ile Lys Thr Thr Pro Tyr Tyr Ile Leu Lys Lys Tyr
           195                 200                 205
   Gln Tyr Trp Gln Arg Ala Val Gly Ser Asn Val Ala Leu Arg Pro His
       210                 215                 220
   Glu Lys Lys Ser Tyr Thr Tyr Glu Trp Gly Thr Glu Ile Asp Gln Lys
   225                 230                 235                 240
   Thr Thr Ile Ile Asn Thr Leu Gly Phe Gln Ile Asn Ile Asp Ser Gly
                   245                 250                 255
   Met Lys Phe Asp Ile Pro Glu Val Gly Gly Gly Thr Asp Glu Ile Lys
               260                 265                 270
   Thr Gln Leu Asn Glu Glu Leu Lys Ile Glu Tyr Ser His Glu Thr Lys
           275                 280                 285
   Ile Met Glu Lys Tyr Gln Glu Gln Ser Glu Ile Asp Asn Pro Thr Asp
   290 295 300
   Gln Ser Met Asn Ser Ile Gly Phe Leu Thr Ile Thr Ser Leu Glu Leu
   305 310 315 320
   Tyr Arg Tyr Asn Gly Ser Glu Ile Arg Ile Met Gln Ile Gln Thr Ser
   325 330 335
   Asp Asn Asp Thr Tyr Asn Val Thr Ser Tyr Pro Asn His Gln Gln Ala
   340 345 350
   Leu Leu
<210> 3
   <211> 659
   <212> PRT
   <213> Bacillus thuringiensis
<400> 3
   Met Ile Arg Met Gly Gly Arg Lys Met Asn Pro Asn Asn Arg Ser Glu
   1               5                   10                  15
   Tyr Asp Thr Ile Lys Val Thr Pro Asn Ser Glu Leu Pro Thr Asn His
               20                  25                  30
   Asn Gln Tyr Pro Leu Ala Asp Asn Pro Asn Ser Thr Leu Glu Glu Leu
           35                  40                  45
   Asn Tyr Lys Glu Phe Leu Arg Met Thr Ala Asp Asn Ser Thr Glu Val
       50                  55                  60
   Leu Asp Ser Ser Thr Val Lys Asp Ala Val Gly Thr Gly Ile Ser Val
   65                  70                  75                  80
   Val Gly Gln Ile Leu Gly Val Val Gly Val Pro Phe Ala Gly Ala Leu
                   85                  90                  95
   Thr Ser Phe Tyr Gln Ser Phe Leu Asn Ala Ile Trp Pro Ser Asp Ala
               100                 105                 110
   Asp Pro Trp Lys Ala Phe Met Ala Gln Val Glu Val Leu Ile Asp Lys
           115                 120                 125
   Lys Ile Glu Glu Tyr Ala Lys Ser Lys Ala Leu Ala Glu Leu Gln Gly
       130                 135                 140
   Leu Gln Asn Asn Phe Glu Asp Tyr Val Asn Ala Leu Asp Ser Trp Lys
   145                 150                 155                 160
   Lys Ala Pro Val Asn Leu Arg Ser Arg Arg Ser Gln Asp Arg Ile Arg
                   165                 170                 175
   Glu Leu Phe Ser Gln Ala Glu Ser His Phe Arg Asn Ser Met Pro Ser
               180                 185                 190
   Phe Ala Val Ser Lys Phe Glu Val Leu Phe Leu Pro Thr Tyr Ala Gln
           195                 200                 205
   Ala Ala Asn Thr His Leu Leu Leu Leu Lys Asp Ala Gln Val Phe Gly
       210                 215                 220
   Glu Glu Trp Gly Tyr Ser Ser Glu Asp Ile Ala Glu Phe Tyr Gln Arg
   225                 230                 235                 240
   Gln Leu Lys Leu Thr Gln Gln Tyr Thr Asp His Cys Val Asn Trp Tyr
                   245                 250                 255
   Asn Val Gly Leu Asn Ser Leu Arg Gly Ser Thr Tyr Asp Ala Trp Val
               260                 265                 270
   Lys Phe Asn Arg Phe Arg Arg Glu Met Thr Leu Thr Val Leu Asp Leu
           275                 280                 285
   Ile Val Leu Phe Pro Phe Tyr Asp Val Arg Leu Tyr Ser Lys Gly Val
       290                 295                 300
   Lys Thr Glu Leu Thr Arg Asp Ile Phe Thr Asp Pro Ile Phe Thr Leu
   305                 310                 315                 320
   Asn Ala Leu Gln Glu Tyr Gly Pro Thr Phe Ser Ser Ile Glu Asn Ser
                   325                 330                 335
   Ile Arg Lys Pro His Leu Phe Asp Tyr Leu Arg Gly Ile Glu Phe His
               340                 345                 350
   Thr Arg Leu Arg Pro Gly Tyr Ser Gly Lys Asp Ser Phe Asn Tyr Trp
           355                 360                 365
   Ser Gly Asn Tyr Val Glu Thr Arg Pro Ser Ile Gly Ser Asn Asp Thr
       370                 375                 380
   Ile Thr Ser Pro Phe Tyr Gly Asp Lys Ser Ile Glu Pro Ile Gln Lys
   385                 390                 395                 400
   Leu Ser Phe Asp Gly Gln Lys Val Tyr Arg Thr Ile Ala Asn Thr Asp
                   405                 410                 415
   Ile Ala Ala Phe Pro Asp Gly Lys Ile Tyr Phe Gly Val Thr Lys Val
               420                 425                 430
   Asp Phe Ser Gln Tyr Asp Asp Gln Lys Asn Glu Thr Ser Thr Gln Thr
           435                 440                 445
   Tyr Asp Ser Lys Arg Tyr Asn Gly Tyr Leu Gly Ala Gln Asp Ser Ile
       450                 455                 460
   Asp Gln Leu Pro Pro Glu Thr Thr Asp Glu Pro Leu Glu Lys Ala Tyr
   465                 470                 475                 480
   Ser His Gln Leu Asn Tyr Ala Glu Cys Phe Leu Met Gln Asp Arg Arg
                   485                 490                 495
   Gly Thr Ile Pro Phe Phe Thr Trp Thr His Arg Ser Val Asp Phe Phe
               500                 505                 510
   Asn Thr Ile Asp Ala Glu Lys Ile Thr Gln Leu Pro Val Val Lys Ala
           515                 520                 525
   Tyr Ala Leu Ser Ser Gly Ala Ser Ile Ile Glu Gly Pro Gly Phe Thr
       530                 535                 540
   Gly Gly Asn Leu Leu Phe Leu Lys Glu Ser Ser Asn Ser Ile Ala Lys
   545                 550                 555                 560
   Phe Lys Val Thr Leu Asn Ser Ala Ala Leu Leu Gln Arg Tyr Arg Val
                   565                 570                 575
   Arg Ile Arg Tyr Ala Ser Thr Thr Asn Leu Arg Leu Phe Val Gln Asn
               580                 585                 590
   Ser Asn Asn Asp Phe Leu Val Ile Tyr Ile Asn Lys Thr Met Asn Ile
           595                 600                 605
   Asp Gly Asp Leu Thr Tyr Gln Thr Phe Asp Phe Ala Thr Ser Asn Ser
       610                 615                 620
   Asn Met Gly Phe Ser Gly Asp Thr Asn Asp Phe Ile Ile Gly Ala Glu
   625                 630                 635                 640
   Ser Phe Val Ser Asn Glu Lys Ile Tyr Ile Asp Lys Ile Glu Phe Ile
                   645                 650                 655
   Pro Val Gln
<210> 4
   <211> 489
   <212> PRT
   <213> Bacillus thuringiensis
<400> 4
   Ser Gln Asp Arg Ile Arg Glu Leu Phe Ser Gln Ala Glu Ser His Phe
   1               5                   10                  15
   Arg Asn Ser Met Pro Ser Phe Ala Val Ser Lys Phe Glu Val Leu Phe
               20                  25                  30
   Leu Pro Thr Tyr Ala Gln Ala Ala Asn Thr His Leu Leu Leu Leu Lys
           35                  40                  45
   Asp Ala Gln Val Phe Gly Glu Glu Trp Gly Tyr Ser Ser Glu Asp Ile
       50                  55                  60
   Ala Glu Phe Tyr Gln Arg Gln Leu Lys Leu Thr Gln Gln Tyr Thr Asp
   65                  70                  75                  80
   His Cys Val Asn Trp Tyr Asn Val Gly Leu Asn Ser Leu Arg Gly Ser
                   85                  90                  95
   Thr Tyr Asp Ala Trp Val Lys Phe Asn Arg Phe Arg Arg Glu Met Thr
               100                 105                 110
   Leu Thr Val Leu Asp Leu Ile Val Leu Phe Pro Phe Tyr Asp Val Arg
           115                 120                 125
   Leu Tyr Ser Lys Gly Val Lys Thr Glu Leu Thr Arg Asp Ile Phe Thr
       130                 135                 140
   Asp Pro Ile Phe Thr Leu Asn Ala Leu Gln Glu Tyr Gly Pro Thr Phe
   145                 150                 155                 160
   Ser Ser Ile Glu Asn Ser Ile Arg Lys Pro His Leu Phe Asp Tyr Leu
                   165                 170                 175
   Arg Gly Ile Glu Phe His Thr Arg Leu Arg Pro Gly Tyr Ser Gly Lys
               180                 185                 190
   Asp Ser Phe Asn Tyr Trp Ser Gly Asn Tyr Val Glu Thr Arg Pro Ser
           195                 200                 205
   Ile Gly Ser Asn Asp Thr Ile Thr Ser Pro Phe Tyr Gly Asp Lys Ser
       210                 215                 220
   Ile Glu Pro Ile Gln Lys Leu Ser Phe Asp Gly Gln Lys Val Tyr Arg
   225                 230                 235                 240
   Thr Ile Ala Asn Thr Asp Ile Ala Ala Phe Pro Asp Gly Lys Ile Tyr
                   245                 250                 255
   Phe Gly Val Thr Lys Val Asp Phe Ser Gln Tyr Asp Asp Gln Lys Asn
               260                 265                 270
   Glu Thr Ser Thr Gln Thr Tyr Asp Ser Lys Arg Tyr Asn Gly Tyr Leu
           275                 280                 285
   Gly Ala Gln Asp Ser Ile Asp Gln Leu Pro Pro Glu Thr Thr Asp Glu
       290                 295                 300
   Pro Leu Glu Lys Ala Tyr Ser His Gln Leu Asn Tyr Ala Glu Cys Phe
   305                 310                 315                 320
   Leu Met Gln Asp Arg Arg Gly Thr Ile Pro Phe Phe Thr Trp Thr His
                   325                 330                 335
   Arg Ser Val Asp Phe Phe Asn Thr Ile Asp Ala Glu Lys Ile Thr Gln
               340                 345                 350
   Leu Pro Val Val Lys Ala Tyr Ala Leu Ser Ser Gly Ala Ser Ile Ile
           355                 360                 365
   Glu Gly Pro Gly Phe Thr Gly Gly Asn Leu Leu Phe Leu Lys Glu Ser
       370                 375                 380
   Ser Asn Ser Ile Ala Lys Phe Lys Val Thr Leu Asn Ser Ala Ala Leu
   385                 390                 395                 400
   Leu Gln Arg Tyr Arg Val Arg Ile Arg Tyr Ala Ser Thr Thr Asn Leu
                   405                 410                 415
   Arg Leu Phe Val Gln Asn Ser Asn Asn Asp Phe Leu Val Ile Tyr Ile
               420                 425                 430
   Asn Lys Thr Met Asn Ile Asp Gly Asp Leu Thr Tyr Gln Thr Phe Asp
           435                 440                 445
   Phe Ala Thr Ser Asn Ser Asn Met Gly Phe Ser Gly Asp Thr Asn Asp
       450                 455                 460
   Phe Ile Ile Gly Ala Glu Ser Phe Val Ser Asn Glu Lys Ile Tyr Ile
   465                 470                 475                 480
   Asp Lys Ile Glu Phe Ile Pro Val Gln
                   485
<210> 5
   <211> 123
   <212> PRT
   <213> Bacillus thuringiensis
<400> 5
   Met Ser Ala Arg Glu Val His Ile Asp Val Asn Asn Lys Thr Gly His
   1               5                   10                  15
   Thr Leu Gln Leu Glu Asp Lys Thr Lys Leu Asp Gly Gly Arg Trp Arg
               20                  25                  30
   Thr Ser Pro Thr Asn Val Ala Asn Asp Gln Ile Lys Thr Phe Val Ala
           35                  40                  45
   Glu Ser Asn Gly Phe Met Thr Gly Thr Glu Gly Thr Ile Tyr Tyr Ser
       50                  55                  60
   Ile Asn Gly Glu Ala Glu Ile Ser Leu Tyr Phe Asp Asn Pro Phe Ala
   65                  70                  75                  80
   Gly Ser Asn Lys Tyr Asp Gly His Ser Asn Lys Ser Gln Tyr Glu Ile
                   85                  90                  95
   Ile Thr Gln Gly Gly Ser Gly Asn Gln Ser His Val Thr Tyr Thr Ile
               100                 105                 110
   Gln Thr Thr Ser Ser Arg Tyr Gly His Lys Ser
           115                 120